# EUROPEAN PATENT APPLICATION

(11) **EP 2 141 167 A1**
(43) Date of publication of application: **06.01.2010**
(21) Application number: 09163520.1
(22) Date of filing: 23.06.2009
(51) Int. Cl.: C07D 477/20

(54) **Process for synthesizing carbapenem using raney nickel**

(30) Priority: 04.07.2008 IT MI20081227
(71) Applicant: ACS DOBFAR S.p.A., 20067 Tribiano (Milano) (IT)
(72) Inventor: Manca, Antonio, 20133 Milano (IT); Monguzzi, Riccardo Ambrogio, 23824 Dorio (LC) (IT)
(74) Representative: Frignoli, Luigi

(57) **Abstract**

Convenient method for obtaining carbapenem by hydrogenation with Raney Nickel, as an alternative to the known catalytic hydrogenation conducted under hydrogen overpressure in the presence of Palladium, starting from corresponding protected intermediates such as p-nitrobenzylesters and with optional suitable protections of any primary and secondary amino functions structurally present.

## Description

The present invention relates to a new process for synthesizing carbapenem of formula (I): 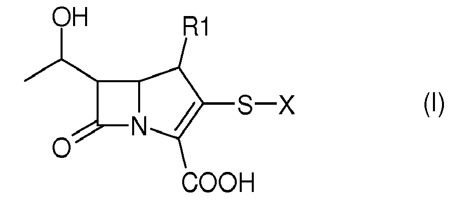 or of a pharmaceutically acceptable salified, prodrug or hydrated form thereof, in which R1 is chosen from the group consisting of H and C1-C6 alkyls, X is chosen from the group consisting of
-CH₂-CH₂-NH-CH=NH, -CH₂-CH₂-NH₂, 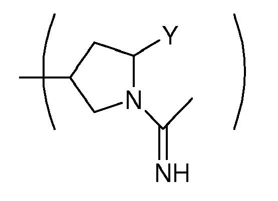 and 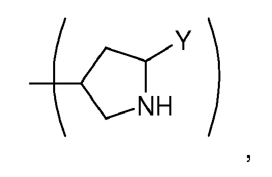 where Y is chosen from the group consisting of 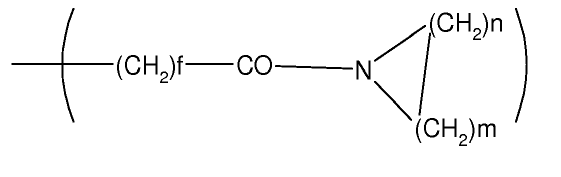 in which f is equal to 0 or 1, while m and n, whole numbers different from 0, are independently chosen to form an azaheterocycle from 3 to 6 carbon atoms, -H, -CN, hydroxyl(C1-C6)alkyls, carbamoyloxy, -OH, substituted or non-substituted linear or branched chain C1-C6 alkyls, C2-C6 alkenyls, C3-C6 cycloalkyls, -R8NHSO₂R6 in which R8 is a C1-C3 alkyl chain, - NHR9-SO₂-R6 in which R9 and R6 are independently chosen from the group consisting of hydrogen or C1-C6 alkyls, -N(R7)₂ where R7 is a C1-C3 alkyl chain, 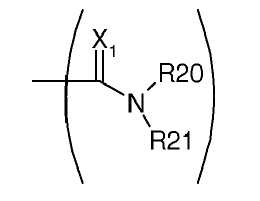 in which X1 is chosen from the group consisting of NH and O while R20 and R21, when independently selected, are chosen from the group comprising H, C1-C5 alkyls, C3-C5 alkenyls, aryl, substituted aryl, substituted aryl carboxy, aralkyls which can be variously substituted with C1-C3 alkyl substituents or monosubstituted with a C1-C5 alkyl or a pyridyl, or R20 and R21, when linked together, are chosen from the group comprising alkylene chains, alkylene chains bridged by a nitrogen atom substituted by a C2-C3 alkyl chain to form a diazaheterocycle, (C1-C5)alkyls-L-G-(C1-C5)alkyls, -NH, -N(L) in which G is O, S, while L are alkyls or alkenyls optionally substituted by from 1 to 3 substituents chosen from the group consisting of 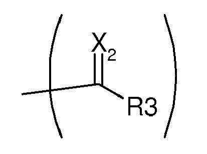 in which X₂ is chosen from the group consisting of O or NH while R3 è chosen from the group of the following heterocycles 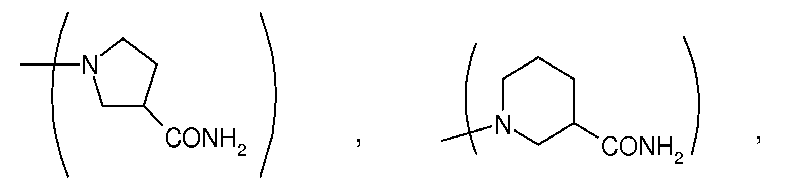 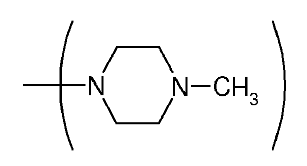 and amines, optionally substituted with 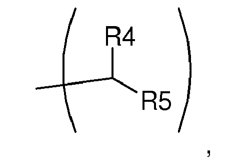 in which R4 and R5 are independently chosen from the group consisting of H, hydroxy(C1-C6)alkyls, CN, amino, carbamoyl, carbamoyl(C1-C6)alkyls, cyano(C1-C6)alkyls, mono- or di-(C1-C6)alkylcarbamoyl, carbamoyloxy, ureide, amino(C1-C6)alkyl, carbamoyloxy(C1-C6)alkyls, mono- or di-(C1-C6)alkylcarbamoyl-(C1-C6)alkyls, ureide(C1-C6)alkyls.

The compounds of formula (I), as described in the known art, are generally obtained from the corresponding intermediates of formula (II) 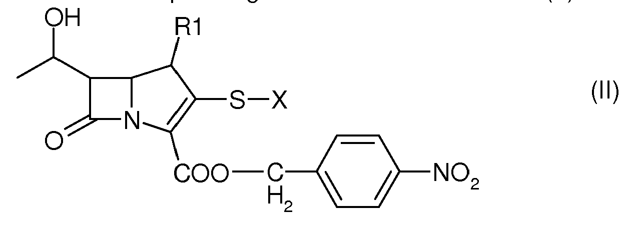 with X and R1 as described above, in which any primary and secondary amino functions present are optionally and suitably protected. This process comprises, as its key passage, a catalytic hydrogenation of the intermediates (II) in the presence of Palladium in a pressure vessel with a hydrogen overpressure much higher than atmospheric.

The use of hydrogen in the presence of Palladium as catalyst, for processes in which transformation of the intermediates (II) into the corresponding compounds (I), or via deprotection of the p-nitrobenzyl ester as in the case of synthesis of meropenem of formula (III), 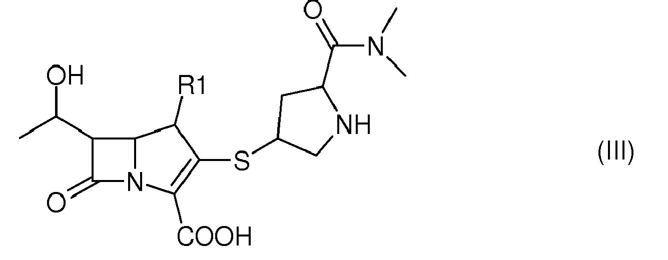 is not free of potential explosion and inflammability dangers as a hydrogen overpressure of generally from 3 to 5 atmospheres is required, using a suitable autoclave.

Autoclave hydrogenation of p-nitrobenzylester, for certain intermediate (II) compounds in which at least one primary or secondary amino function without suitable protection, generally of oxycarbonyl or allyloxycarbonyl or p-nitrobenzyloxycarbonyl type, additionally requires in-process pH monitoring by simultaneous addition of gaseous carbon dioxide for the necessary pH lowering, as described in the Journal of Organic Chemistry 2005, 70, 7479-7487 for the synthesis of ertapenem of formula (IV) 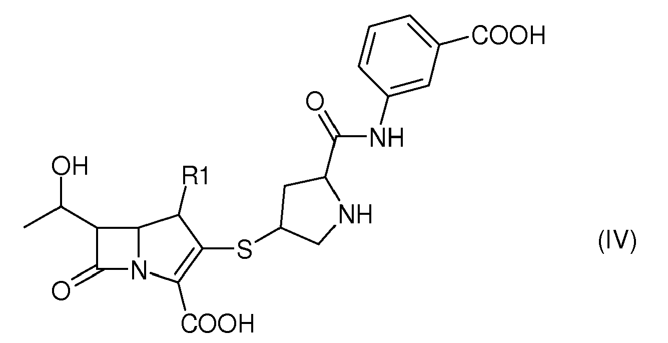 starting from the compound (II), ertapenem p-nitrobenzylester, where X equals 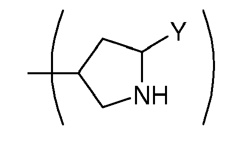 in which Y is

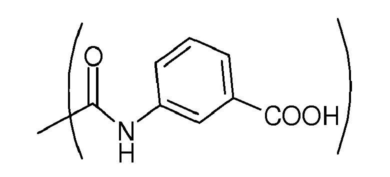

Likewise, the compound of formula (I), where R1 is hydrogen and X is - CH2-CH2-NH2 (thienamycin), can be obtained starting from the corresponding intermediate (II) not protected at the primary amino group, with X evidently equal to -CH2-CH2-NH2 (thienamycin p-nitrobenzylester), only via a hydrogenation process having the same operating arrangement as described for ertapenem.

This laborious operating arrangement makes it necessary to use a particular autoclave equipped with a suitable probe for in-process pH monitoring and a valve for adding gaseous carbon dioxide flanking that dedicated to hydrogen feed.

In contrast, the present inventors have surprisingly verified that the p-nitrobenzylesters of formula (II), even those presenting amino groups without the said oxycarbonyl protection, are advantageously deprotected by the use of Raney Nickel at hydrogen pressures less than 1.3 atm, i.e. at the operating pressures of a common process reactor with a rupture disc set for overpressures not exceeding 0.45 atm, i.e. using a solution of Raney Nickel previously saturated with hydrogen, without it then being necessary to use an autoclave for conducting the hydrogen overpressure reaction. The starting product is dissolved in an aqueous buffer at pH between 4.0 and 8.0, to which a water miscible or immiscible protic or aprotic organic solvent has been optionally added; an aqueous suspension of Raney Nickel is then added under vigorous agitation, in a quantity generally between 70 ml and 350 ml per molar equivalent of p-nitrobenzyl groups present in the protected substrate, leaving it to react for 1-6 hours at a temperature between +1ºC and +50ºC. The product, deprotected and hence obtained as carboxylate, is isolated from the resultant aqueous solution on termination of the reaction. After eliminating the metal residues by filtration or decantation, the aqueous solution in which the product is dissolved is separated from the water-immiscible organic solvent possibly used in the process, after which the salts present in the aqueous solution are eliminated by chromatography, ultrafiltration or reverse osmosis: the product can be obtained from the obtained aqueous solution by precipitation with a water-miscible organic solvent or by lyophilization. Alternatively a crude product can be obtained from the aqueous solution rich in saline residues by lyophilisation with subsequent dissolving of the obtained product in a suitable organic solvent, in which the co-lyophilized saline residues are eliminated by filtration and the product then obtained by evaporating the previously filtered solution in which it is dissolved. The product can also be isolated by precipitation from the aqueous solution in which it is dissolved: the precipitation is pursued by adding a suitable organic solvent miscible with said aqueous solution. Finally, adding a co-solvent to an aqueous solution of the product obtained on termination of the reaction, after filtering off the metal residues and when the process is optimized, enables selective precipitation of said product while leaving in solution the saline species used to form the aforesaid reaction buffer, generally in the case in which this buffer species essentially consists of an organic salt. The intermediates included in the compounds of formula (II), such as those represented by formulas (Ilbis and Ilter), 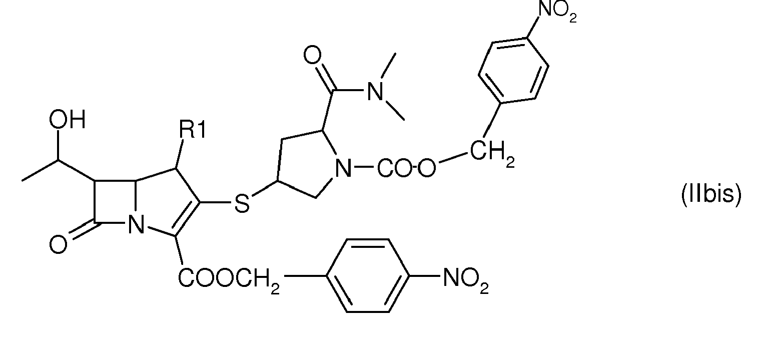 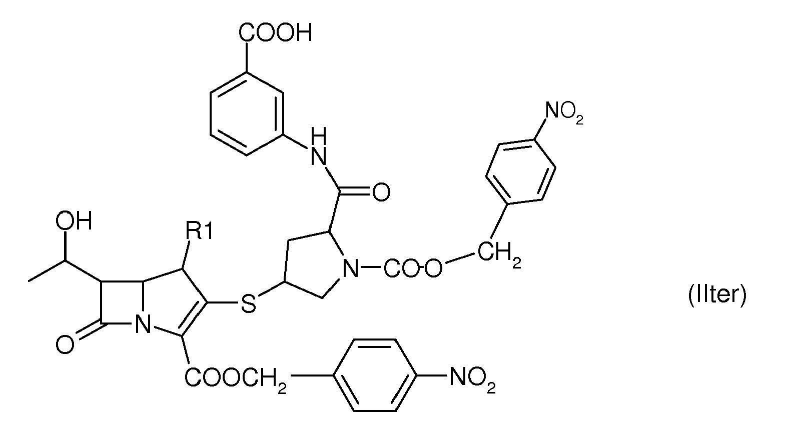 which present the p-nitrobenzyloxycarbonyl protection on at least one of the primary or secondary amino groups present, are directly converted to the corresponding carbapenam included in formula (I) in the presence of Raney Nickel in aqueous suspension, with hydrogen pressures less than 1.3 atm without isolating any intermediate product.

In contrast, the intermediates (II), characterised by other suitable protections different from p-nitrobenzyloxycarbonyl for at least one of any primary or secondary amino groups present, require a further process of deprotecting said amino functions by known methods, to obtain the corresponding products of formula (I), whether the p-nitrobenzylester deprotection is conducted by the known method consisting of hydrogenation in autoclave with a hydrogen overpressure greater than 3 atm or by the new method illustrated in the following invention; in particular, following deprotection of the p-nitrobenzylester of the intermediate (II), the optional allyloxycarbonyl protections, for any primary or secondary amino groups present in said intermediate, are released by the known use of palladium(O)tetrakistriphenylphosphine in the presence either of triphenylphosphine or, preferably, of aniline.

Proposed experimental examples, which are non-limiting for the invention, are described below.

### EXAMPLE 1

### Preparation of carbapenem (III) - Meropenem

A buffer solution is prepared by dissolving 20 g of disodium hydrogenphosphate in 150 ml of demineralised water. This is buffered at pH 6.5 with 85% aqueous phosphoric acid and 10ml of an aqueous suspension of Raney Nickel are added. 10 g of the compound (Ilbis) previously dissolved in 150 ml of ethyl acetate are added at a temperature of +20ºC, and the mixture left to react for 5 hours in a hydrogen atmosphere (1.0 atm pressure in a glass reactor). It is cooled to +5ºC and the insolubles filtered off. The filtrate is washed with 20 ml of demineralized water and the phases separated. The underlying aqueous phase is evaporated. 400 g of HP-20L resin are added to the evaporated aqueous solution. It is left under agitation for 20 minutes, filtered, washed with water, initially with 1200 ml, then with 800 ml, then with 500 ml and finally with 500 ml. The product, fixed on the resin washed in this manner with water, is recovered by extraction with aqueous acetone, by suspending the resin initially in 400 ml of a 10% acetone solution, then twice with 400ml of 15% acetone, and finally with 400 ml of 25% acetone. The aqueous acetone solutions obtained are pooled and the acetone evaporated. The resultant aqueous solution is concentrated by reverse osmosis to 700 ml, which is finally lyophilized to obtain 4.5 g of product (III).

### EXAMPLE 2

### Preparation of carbapenem (IV) - Ertapenem.

The intermediate ertapenem p-nitrobenzylester, included in the compounds of formula (II) from which carbapenem (IV) is obtained, is synthesized in accordance with the teachings of the Journal of Organic Chemistry 2005, 70, 7479-7487 on page 7486, then isolated.

8.5 g of (4R,5S,6S,8R)-3-[(diphenoxyphosphinyl) oxy]-6-(hydroxyethyl)-4-methyl-7-oxo-1-azabicyclo[3.2.0]hept-2-ene-2-carboxylate of (4-nitrophenyl)methyl are dissolved at 0ºC in a nitrogen atmosphere in a solution of 63.2 ml of N-ethylpyrrolidone and 3.30 ml of water. 0.035 ml of tri-n-butylphosphine are added, followed by 4.29 g of (2CS-cis)-3-[(4-mercapto-2-pyrrolidinyl carbonyl]amino] benzoic acid monohydrochloride.

The mixture is agitated for 15 minutes and cooled to -55ºC/-60ºC, then while maintaining the temperature less than -50ºC, 5.99 ml of tetramethylguanidine are added under vigorous agitation. The mixture is agitated for 1 hour at -50ºC/-55°C then, while reaction is continued for 1 further hour at -40°C. The temperature is lowered to -50°C and, maintaining the temperature below -40ºC, 2.14 ml of glacial acetic acid are added. The temperature is raised to 0ºC and 80 ml of n-butanol are added, the solution obtained being poured into 1000 ml of n-hexane. The precipitate obtained is filtered off and washed in portions with 100 ml of n-hexane, then dried at +25ºC under reduced pressure, to obtain 7.0 g of ertapenem p-nitrobenzylester.

The ertapenem p-nitrobenzylester obtained is dissolved in 175 ml of ethyl acetate. 175 ml of a 0.6 M solution of sodium dihydrogenphosphate adjusted to pH 6.2 are added to the solution obtained.

4.0 ml of an aqueous suspension of Raney Nickel are added at a temperature of +20ºC. The mixture is agitated for 5 hours at +20ºC under a nitrogen atmosphere (1 atm) in a suitable glass reactor.

The metal residues are filtered off and the filtrate washed with 20 ml of water. The phases are separated. The separated underlying aqueous phase is then lyophilized.

The lyophilized product is suspended in 350 ml of methanol and agitated for 10 minutes. The insolubles in the form of inorganic salts are filtered off.

The solution is evaporated at +30ºC until a dense oil is obtained. 0.7 ml of water and 70 ml of ethanol are added and the mixture cooled to -10ºC, slowly agitating at this temperature for 5 hours. It is filtered and washed with 35 ml of ethanol in two portions. After drying at +25ºC under reduced pressure, 5.25 g of product (IV), ertapenem sodium, are obtained.

## Claims

1. A process for synthesizing carbapenem of formula (I): 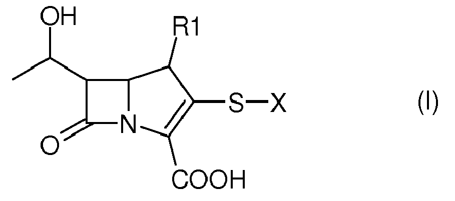 or of a pharmaceutically acceptable salified, prodrug or hydrated form thereof, in which R1 is chosen from the group consisting of H and C1-C6 alkyls, X is chosen from the group consisting of
-CH₂-CH₂-NH-CH=NH, -CH₂-CH₂-NH₂, 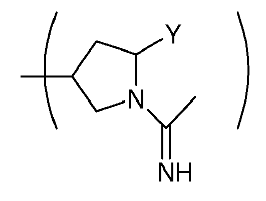 and 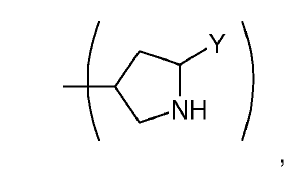 where Y is chosen from the group consisting of 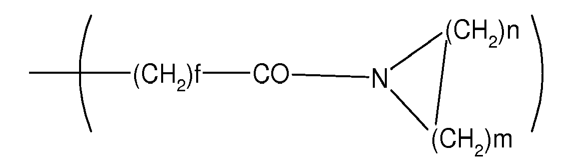 in which f is equal to 0 or 1, while m and n, whole numbers different from 0, are independently chosen to form an azaheterocycle from 3 to 6 carbon atoms, -H, -CN, hydroxy(C1-C6)alkyls, carbamoyloxy, -OH, substituted or non-substituted linear or branched chain C1-C6 alkyls, C2-C6 alkenyls, C3-C6 cycloalkyls, -R8NHSO₂R6 in which R8 is a C1-C3 alkyl chain, - NHR9-SO₂-R6 in which R9 and R6 are independently chosen from the group consisting of hydrogen or C1-C6 alkyls, -N(R7)₂ where R7 is a C1-C3 alkyl chain, 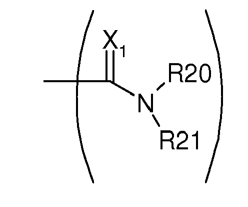 in which X1 is chosen from the group consisting of NH and O while R20 and R21, when independently selected, are chosen from the group comprising H, C1-C5 alkyls, C3-C5 alkenyls, aryl, substituted aryl, substituted aryl carboxy, aralkyls which can be variously substituted with C1-C3 alkyl substituents or monosubstituted with a C1-C5 alkyl or a pyridyl, or R20 and R21, when linked together, are chosen from the group comprising alkylene chains, alkylene chains bridged by a nitrogen atom substituted by a C2-C3 alkyl chain to form a diazaheterocycle, (C1-C5)alkyls-L-G-(C1-C5)alkyls, -NH, -N(L) in which G is O, S, while L are alkyls or alkenyls optionally substituted by from 1 to 3 substituents chosen from the group consisting of 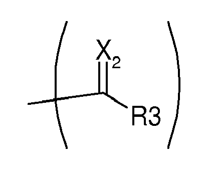 in which X₂ è chosen from the group consisting of O or NH while R3 è chosen from the group of the following azaheterocycles 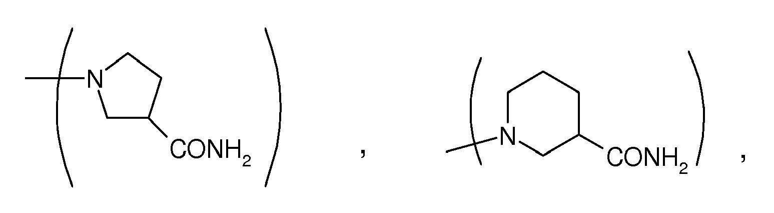 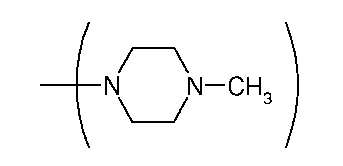 and amines, optionally substituted with 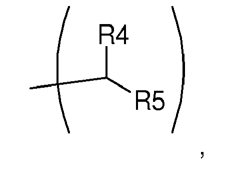 in which R4 and R5 are independently chosen from the group consisting of H, hydroxy(C1-C6)alkyls, CN, amino, carbamoyl, carbamoyl(C1-C6)alkyls, cyano(C1-C6)alkyls, mono- or di-(C1-C6)alkylcarbamoyl, carbamoyloxy, ureide, amino(C1-C6)alkyl, carbamoyloxy(C1-C6)alkyls, mono- or di-(C1-C6)alkylcarbamoyl-(C1-C6)alkyls, ureide(C1-C6)alkyls, starting from the corresponding intermediates of formula (II) 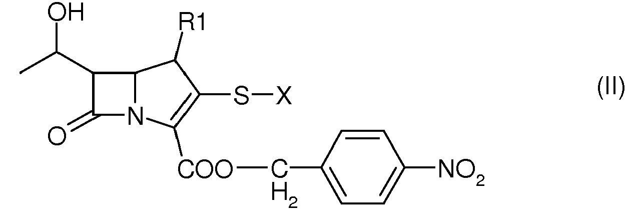 in which X and R1 have the meanings indicated for the compounds of formula (I), where any primary and secondary amino functions present can be optionally protected with suitable protective groups, by deprotecting the p-nitrobenzyl ester of the intermediate (II) with Raney Nickel in water miscible or immiscible protic or aprotic organic solvents, in the presence of hydrogen which may even have been simply preabsorbed on the Raney Nickel.

2. A process as claimed in claim 1, wherein said optional suitable protective groups, which may be present for at least one of the primary and secondary functions of the intermediates of formulas (II), are chosen from the group consisting of p-nitrobenzyloxycarbonyl and allyloxycarbonyl.

3. A process as claimed in claim 1 for obtaining meropenem and ertapenem, compounds comprised in formula (I), of formula (III) and (IV) respectively
